(19) **Europäisches Patentamt European Patent Office Office européen des brevets**

(11) **EP 3 589 186 B1**

(12) **EUROPEAN PATENT SPECIFICATION**

(45) Date of publication and mention of the grant of the patent:
**15.07.2020 Bulletin 2020/29**

(21) Application number: **18708116.1**

(22) Date of filing: **28.02.2018**

(51) Int Cl.:
*A61B 5/00* (2006.01)　　　*A61B 5/0215* (2006.01)
*A61B 5/027* (2006.01)　　　*A61M 25/00* (2006.01)

(86) International application number:
**PCT/EP2018/054959**

(87) International publication number:
**WO 2018/158331 (07.09.2018 Gazette 2018/36)**

(54) **INTRAVASCULAR BLOOD FLOW MEASUREMENT USING DIFFERENTIAL PRESSURE PRINCIPLE AND AN EXPANDING FLOW SENSOR**

INTRAVASKULÄRE BLUTFLUSSMESSUNG

MESURE DE DÉBIT SANGUIN INTRAVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2017 EP 17158386**

(43) Date of publication of application:
**08.01.2020 Bulletin 2020/02**

(73) Proprietor: **Koninklijke Philips N.V. 5656 AG Eindhoven (NL)**

(72) Inventors:
• MÜLLER, Manfred
  **5656 AE Eindhoven (NL)**
• HENDRIKS, Cornelis, Petrus
  **5656 AE Eindhoven (NL)**

(74) Representative: **de Haan, Poul Erik Philips International B.V. Philips Intellectual Property & Standards High Tech Campus 5 5656 AE Eindhoven (NL)**

(56) References cited:
**US-A- 5 178 153**

**Description**

FIELD OF THE INVENTION

The present invention relates to an intravascular blood flow measurement device, to a blood flow measurement system, to a method for controlling operation of an intravascular blood flow measurement device, to a method for determination of a blood flow quantity inside a blood vessel of a living being and to a computer program.

BACKGROUND OF THE INVENTION

Intravascular flow measurements find important clinical applications in cardiology and in medical examinations of peripheral arteries. Measuring coronary flow reserve (CFR) is a recognized way to determine whether a stenosis in coronary arteries is flow limiting. CFR involves measuring flow in a stenosed artery at rest and during hyperemia. Flow measurements are considered particularly suitable to characterize a stenosis. However, measuring flow is challenging compared to measuring pressure. For example, Doppler ultrasound measurements require a precise and time-consuming placement of the sensor wire in the blood vessel.

US 2015/0313478 A1 describes computer-based methods, devices, and systems suitable for performing intravascular data analysis and measurement of various types of data such as pressure and flow data. The disclosure relates to probes and methods suitable for determining an event in a cardiac cycle such as flow threshold, such as a peak flow, a fraction thereof, other intravascular parameters or a point in time during which peak flow or a change in one of the parameters occurs. A probe includes one or more of a pressure sensor, a resistor, a flow sensor and can be used to generate diagnostic data based upon measured intravascular and other parameters. Methods and systems suitable for determining a coronary flow reserve value in response to one or more of intravascular pressure and flow data or data otherwise correlated therewith are described.

US5178153 describes an optical fiber fluid flow device for in vivo determination of blood flow in arteries based on the Venturi effect.

SUMMARY OF THE INVENTION

It is an object of the present invention to provide a simpler and thus more cost effective alternative to existing intravascular blood flow measurement devices for determining a flow rate of blood inside a blood vessel.

According to a first aspect of the present invention, an intravascular blood flow measurement device is provided. The intravascular blood flow measurement device comprises:

- a body suitable for placement inside a blood vessel of a living being and extending along a longitudinal direction;
- an expandable body section of the body, having a longitudinal extension in the longitudinal direction, and a cross-sectional area perpendicular to the longitudinal direction that is gradually changing in longitudinal direction and that is controllably and reversibly expandable to assume a respective one of at least two different predetermined expanded sizes associated with at least two respective values of an expanded cross sectional area of the expandable body section;
- a controllable actuator arranged and configured to set the expandable body section to one of the at least two sizes; and
- a pressure sensor unit comprising at least one pressure sensor arranged on the body within the longitudinal extension of the expandable body section, the pressure sensor unit being configured to provide, for each of at least three values of cross sectional area of the body, including the at least two values of the expanded cross-sectional area of the expandable body section, a respective sequence of pressure signals indicative of a pressure of blood flow in the blood vessel over a predetermined measuring time span defined with respect to a heart cycle period.

The intravascular blood flow measurement device of the first aspect of the present invention implements a novel way to measure intravascular blood flow involving a measurement of a drop in pressure inside an artificial variable stenosis. The expandable body section of the body having variable, but known cross-sections acts as a variable choke. The pressure sensor unit with at least one pressure sensor arranged on the body inside the choke area, that is, within the longitudinal extension of the expandable body section, measures the blood pressure over a predetermined measuring time span defined with respect to a heart cycle period. The pressure measurement is performed for each of at least three values of cross sectional area of the body. These at least three values include the at least two values of the expanded cross-sectional area of the expandable body section.

To this end, the expandable body section is controllably and reversibly expandable to assume different predetermined expanded sizes associated with respective values of an expanded cross sectional area of the expandable body section. The size is changeable by controlled expansion or contraction of the expandable body section.

The present invention makes use of the recognition of the inventors that a pressure drop across the artificial stenosis caused by the expandable body section of the body is mostly caused by turbulent flow in the divergent section of the stenosis. Inside the artificial stenosis created by the expandable body section there is a pressure drop due to the Bernoulli/Venturi effect. For the blood has to flow faster through the constricted section. This pressure drop is used to according to the present invention to determine the flow velocity. It is pointed out that the present invention does not consider placing a measurement device inside a natural stenosis of a patient, because the measurement device will block the blood flow even more. The intravascular blood flow measurement device of the present invention is advantageous in this regard in that it provides an artificial stenosis that can be controlled to be not flow-limiting. In operation, thus, for investigating a natural stenosis in a patient, the intravascular blood flow measurement device is suitably placed proximal of the stenosis to be investigated.

Values of a blood flow quantity such as a blood flow velocity can then be determined using the measured pressure values at the different values of the cross sectional area provided by operating the intravascular blood flow measurement device. This will be explained in more detail further below in the context of the description of the blood flow measurement device of the second aspect of the invention.

In the following, embodiments of the intravascular blood flow measurement device of the first aspect of the present invention are presented.

The intravascular blood flow measurement device of some embodiments is provided in the form of an add-on unit that can be mechanically and electrically connected with or disconnected from a catheter device or guidewire. In other embodiments, the intravascular blood flow measurement device forms an integral part of such catheter device, preferably a microcatheter or guidewire.

The body of the intravascular blood flow measurement device, including the expandable body section, preferably has a stream-lined shape for maintaining a laminar blood flow inside the blood vessel, preferably along the longitudinal extension of the expandable body section. While the pressure drop across a stenosis does involve a turbulent flow in the divergent section downstream of the stenosis, it is advantageous to maintain a laminar flow to before and especially inside the narrowing itself so that Bernoulli's law applies.

To achieve that, the expandable body section suitably has a cross-sectional area value that is gradually changing along longitudinal direction before and after reaching a maximum cross-sectional area value. This applies to all sizes of the expandable body section that can be selected for operation during performance of a flow measurement. This way, turbulences in the blood flow due to the presence of the intravascular blood flow measurement device in the blood vessel can be avoided, which improves the quality of the flow determination.

The expandable body section can be implemented in different ways. The expansion in size and the reverse process of contraction can be effected by any suitable increase or decrease in shape or volume of the expandable body section. In some embodiments, the change in shape is combined with a change in volume of the expandable body section. In other embodiments, the change in size is implemented by only a change in shape while the volume of the expandable body section remains unchanged. In some other embodiments, despite an increase in size, the volume of the expandable body section is even decreased in the course of expansion, and increased in the course of contraction. What matters is the ability of the expandable body section to form a variable artificial stenosis inside the blood vessel.

In some embodiments of the intravascular blood flow measurement device, the expansion and contraction of the expandable body section is achieved using an electro-active polymer (EAP). As is well known per se, EAPs exhibit a change in size or shape when stimulated by a suitable electric signal. The electro-active polymer comprised by the expandable body section of the subject embodiment is configured to controllably change its size by changing its shape or volume in response to receiving an electrical expansion-control signal. The change in size thus may involve a change in volume or a change in shape of the EAP, or a change in shape that at the same time involves a change in volume within a range that can be predetermined by material selection and a suitably designed shape of the expandable body section implemented this way. A non-limiting list of suitable EAPs that are used in different embodiments include piezoelectric polymers, electromechanical polymers, relaxor ferroelectric polymers, electrostrictive polymers, dielectric elastomers, liquid crystal elastomers, conjugated polymers, ionic polymer-metal composites, ionic gels, polymer gels, etc.

In other embodiments, the expansion and contraction of the expandable body section is achieved by using an inflatable balloon that thus forms a volume-expandable body section. In these embodiments, the inflatable balloon is preferably configured to controllably change its volume within a predetermined volume range. The volume range can be determined by a suitably designed shape of the volume-expandable body section implemented this way, and by selection a suitable material as well as a suitable pressure range for driving a volume expansion using a pressurized fluid or gas. The controllable actuator is in these embodiments preferably configured to controllably pump a pressurized fluid or gas into or out of the inflatable balloon, depending on whether an expansion or reduction of the volume is currently desired.

The number of different sizes to be set for performing the blood flow determination according to the concept of the present invention differs in different embodiments. It depends on the number and location of the pressure sensors of the pressure sensor unit.

In one embodiment, the pressure sensor unit comprises at least two pressure sensors, wherein at least one of the pressure sensors is arranged on the body outside the longitudinal extension of the expandable body section. A particularly simple configuration according to this embodiment thus has a first pressure sensor arranged on the body within the longitudinal extension of the expandable body section, and a second pressure sensor arranged on the body outside the longitudinal extension of the expandable body section. The at least two sensors thus build a differential pressure sensor, and since a reference pressure is additionally measured at a position of the intravascular blood flow measurement device that has a fixed cross sectional value (i.e. the pressure sensor arranged outside the longitudinal extension of the expandable body section), these embodiments are particularly robust against heart beat to heart beat variations in the pressure of the blood flow. Furthermore, in this configuration, the required minimum number of size settings of the expandable body section required is reduced to two, for providing two values of the expanded cross sectional area of the expandable body section for pressure measurement using the first pressure sensor. The cross-sectional area of the body outside the longitudinal extension of the expandable body section provides for the third value of cross sectional area of the body, for a pressure measurement using the second pressure sensor, as long as the value cross sectional area of the body outside the longitudinal extension of the expandable body section is different from any of the expanded cross sectional area values of the expandable body section. This way, the minimum total number of three values of cross sectional area of the body (which include the at least two values of the expanded cross-sectional area of the expandable body section) is achieved with a minimum of size changes, thus achieving a particularly fast measurement. In other embodiments that employ a single pressure sensor arranged on the body within the longitudinal extension of the expandable body section, the minimum number of three values of cross sectional area of body is achieved by setting the expandable body section to three different expanded sizes.

The pressure sensor(s) provide(s) respective sequences of pressure signals indicative of a pressure of blood flow in the blood vessel over a predetermined measuring time span defined with respect to a heart cycle period. Preferably, different measuring time spans are distributed over a full heart cycle, which allows determining a flow profile over the heart cycle.

In other embodiments, a larger number of cross sectional area values of the body is employed to achieve further advantages, as will be described further below. This larger number of four or more measurements can be achieved by either using a single pressure sensor, or by using two or more pressure sensors in a configuration described above, wherein different pressure sensors outside the longitudinal extension of the expandable body section can be associated with different local cross sectional area values of the body at the location of the respective pressure sensors.

The design of the intravascular blood flow measurement device can be implemented in different variants. In a preferred variant, the intravascular blood flow measurement device comprises a non-expandable body having a fixed cross sectional area value along its longitudinal extension in a distal end section, a proximal end section, and in a middle section between the distal and proximal end sections. The expandable body section is preferably arranged in the middle section of the non-expandable body. Variants having a non-expandable body with different fixed cross sectional area values along its longitudinal extension, preferably exhibit such variations in different subsections of the distal end section.

According to a second aspect of the present invention, a blood flow measurement system for measuring a blood flow quantity inside a blood vessel of a living being is provided. The blood flow measurement system comprises:

- an intravascular blood flow measurement device according to the first aspect of the present invention or one of its embodiments;
- a control unit configured to sequentially provide to the controllable actuator a size control signal indicative of a respective one of the at least two pre-determined sizes to be assumed by the expandable body section; and
- a flow determination unit, which is configured to receive the pressure signals provided by the pressure sensor unit and, using the at least three values of cross sectional area of the body, the pressure signals associated with the at least three values of cross sectional area of the body and taken at respective measuring times corresponding to an identical phase of the heart cycle, and a known value of a density of blood, to calculate a value of a blood flow quantity inside the blood vessel.

In addition to the features of the intravascular blood flow measurement device of the first aspect of the invention, the blood flow measurement system of the second aspect includes control and evaluation functionality implemented in the control unit and in the flow determination unit.

The control unit and the flow determination unit can be provided in the form of electronic chip products having small size, and either of them or both can be located inside the intravascular blood flow measurement device, in particular the body, according to the specific design requirements of an application case.

Also, either the control unit or the flow determination unit or both may be implemented in integrated form as an integral part of a catheter device for intravascular insertion and operation. Thus, one embodiment of the present invention is formed by an intravascular catheter, comprising the control unit and the flow determination unit. These two units can for instance be arranged at a proximal end of the catheter device. In some embodiments the catheter is preferably a microcatheter.

When implemented as a respective unit of an external control and/or flow determination device that is not for intravascular insertion, the control unit and/or the flow determination unit can be provided in the form of a computer, which is configured to be in communicative connection with the intravascular blood flow measurement device during intravascular operation thereof.

The communicative connection is in some embodiments achieved by communicating via a patch cable connecting the computer with the intravascular blood flow measurement device. In other embodiments, the communicative connection is achieved over an air interface using a wireless communication channel according to any radio communication protocol such as any of the IEEE 802.11 standards, Bluetooth, infrared-based communication protocols, etc.

In some embodiments of the blood flow measurement system that offer a technologically simpler implementation, the pressure sensor unit comprises only one pressure sensor. In these embodiments, the control unit is configured to provide to the controllable actuator a size control signal indicative of a respective one of the at least three predetermined sizes to be assumed by the expandable body section.

In the following, embodiments having a particularly advantageous implementation of the flow determination unit will be described. In one embodiment, the flow determination unit is configured to solve a system of two or more equations, using Bernoulli's law, the at least three values of cross sectional area of the body, the associated sequences of pressure signals, and a known value of a density of blood, and to determine a value of the cross sectional area of the blood vessel and a value of a volume flow velocity at a respective value of cross sectional area. From Bernoulli's law,

$$p + \frac{\rho v^2}{2} = const. \tag{1}$$

it follows that for two different pressure values $p_0$ and $p_i$ and two corresponding different flow velocities $v_0$ and $v_i$ in a medium of density $\rho$ the following equation holds:

$$p_0 - p_i = \frac{\rho}{2}\left(v_i^2 - v_0^2\right) \tag{2}$$

Preferably, therefore, the flow determination unit is configured to solve the system of two equations, each equation having the form

$$p_i = \frac{v_0^2 \rho}{2}\left(1 - \left(\frac{A - A_0}{A - A_i}\right)^2\right) + p_0, \tag{3}.$$

The symbols in this equation have the following meanings:

$A_0$ is a first of the predetermined values of cross sectional area of the body;
$A_i$ is a respective second of the predetermined values of cross sectional area of the body, wherein in the two equations two different predetermined values of cross sectional area are used;
$A$ is a value (to be determined) of a cross sectional area of the blood vessel;
$p_0$ is a first pressure value associated with the respective first value of cross sectional area $A_0$, taken at the respective measuring time corresponding to the identical phase of the heart cycle;
$p_i$ is a second pressure value associated with the respective value of cross sectional area $A_i$, taken at the respective measuring time corresponding to the identical phase of the heart cycle, wherein in the two equations two different measured values of pressure are used;
$\rho$ is the density of the blood;
$v_0$ is the volume flow velocity, to be determined, at the value of cross sectional area $A_0$.

It is noted that the indices 0 and i are arbitrarily allocatable to the different cross sectional area values of the expandable body section and the associated measured pressure values.

Preferably, the flow determination unit is configured to determine a value of a flow rate $Q$ as the blood flow quantity, by calculating

$$(A - A_0)v_0 = Q \text{ or } (A - Ai)v_i = Q \tag{4}.$$

In further advantageous embodiments using a larger number of pressure measurements than the minimum number required, it is possible to additionally determine whether the intravascular blood flow measurement device, as operated, forms an undesirably strong obstacle within the blood vessel. While typically pressure drops at such a device are small and it is unlikely to be limiting the blood flow, a test of the validity of this assumption in a given measurement setting can be made by using at least four measurements. In this embodiment,

- the expandable body section is controllably and reversibly expandable to assume at least three different predetermined expanded sizes associated with at least three respective values of an expanded cross sectional area of the expandable body section;
- the pressure sensor unit is configured to provide, for each of at least four values of cross sectional area of the body, including the at least three values of the expanded cross-sectional area of the expandable body section, the respective sequence of pressure signals; and
- the flow determination unit is configured to determine, using a predetermined fitting model, calibration curve parameters fitting pressure value determined from the received pressure signals as a function of cross-sectional area of the body and to determine, using the pressure values and the associated values of the cross-sectional area of the body, a fit-error measure, to compare the fit-error measure with a pre-determined error threshold value, and, in case the fit-error measure exceeds the error threshold value, to provide an error output signal indicative thereof.

As an example, equation (3) will only fit four or more data points well if the blood flow is not limited. As a result the accuracy of the fit, as for example expressed by the mean squared error of the fit with respect to the measured data points, can be used as the fit-error measure. This way, one can detect if the device itself is reducing the flow. This fit-error measure advantageously also detects cases where variations between heart cycles, i.e., an inaccuracy in the phase of the heart cycle used for measurement and evaluation, distorts the flow value determination.

This embodiment can be extended by providing a feedback to the measurement operation. In one such embodiment, the control unit is configured, in response to receiving the error output signal, to determine and provide to the controllable actuator corrected size control signals indicative of at least two corrected size values within a corrected size value range having a smaller corrected maximum size value in comparison with a maximum size value of the expandable body section set by the control unit before receiving the error output signal. This embodiment works particularly well if the size values are selectable from a predetermined continuous size range, allowing a fine-tuning of the size of the expandable body section of the intravascular blood flow measurement device.

Given such a feedback loop, in case the determined fit quality indicates no flow limitation, an increase the expansion is suitably performed in a similar manner, until flow limiting is detected based on the fit-error measure. This way, the described feedback loop can be used to maximize a signal-to-noise ratio of the flow measurement while making sure that the blood flow is not limited by the device itself.

In addition, or alternatively, some of these embodiments comprise a user interface that is interface configured to receive the error output signal and, upon receiving the error output signal, to provide output information to a user, the output information being indicative of a blood flow limitation induced by the intravascular blood flow measurement device exceeding a predetermined maximum blood flow limitation amount in the blood vessel.

The determined value of blood flow is typically relevant in cases where the presence of the intravascular blood flow measurement device does not exceedingly limit the blood flow, when compared to the blood flow in the vessel without the intravascular blood flow measurement device inserted. These embodiments thus allow an interaction with a user and are suitably configured to aid the user in assessing the relevancy of the calculated blood flow quantity.

According to a third aspect of the present invention, a method for controlling operation of an intravascular blood flow measurement device according to the first aspect is presented. The method comprises:

- sequentially providing to the controllable actuator of the intravascular blood flow measurement device a size control signal indicative of a respective one of at least two predetermined expanded sizes to be assumed by the expandable body section of the body of the intravascular blood flow measurement device; and
- controlling provision, by a pressure sensor unit which comprises at least one pressure sensor arranged on the body of the intravascular blood flow measurement device within a longitudinal extension of the expandable body section, for each of at least three values of cross sectional area of the body, including the at least two values of the expanded cross-sectional area of the expandable body section, of respective sequence of pressure signals indicative of a pressure of blood flow in the blood vessel over a predetermined measuring time span defined with respect to a heart cycle period.

The method shares the advantages of the blood flow measurement system of the second aspect of the invention, and thus also the advantages described in the context of the description of the intravascular blood flow measurement device of the first aspect of the invention.

According to a forth aspect, a method for determination of a blood flow quantity inside a blood vessel of a living being is presented. The method comprises:

- performing a method according to the third aspect of the invention; and
- calculating a value of a blood flow quantity inside the blood vessel, using the at least three values of cross sectional area of the body, the pressure signals associated with the at least three values of cross sectional area of the body and taken at respective measuring times corresponding to an identical phase of the heart cycle, and a known value of a density of blood

The method thus shares the advantages of the method for controlling operation of an intravascular blood flow measurement device of the third aspect.

In one embodiment of the method of the forth aspect, calculating a value of a blood flow quantity inside the blood vessel comprises:

- using Bernoulli's law, the at least three values of cross sectional area of the body, the associated sequences of pressure signals, and a known value of a density of blood, solving a system of two or more equations, each equation having the form of equation (3). In this embodiment, determining the blood flow quantity preferably comprises determining a value of a flow rate by a calculation according to equation (4).

A fifth aspect of the present invention is formed by a computer program comprising executable code for executing a method according to the third or fourth aspect of the invention when executed by a processor of a computer.

Other embodiments of the present invention are thus formed by an intravascular catheter device comprising an intravascular blood flow sensor according to the first aspect of the invention.

It shall be understood that the intravascular blood flow measurement device of claim 1, the blood flow measurement system of claim 7, method for controlling operation of an intravascular blood flow measurement device of claim 12, the method for determination of a blood flow quantity inside a blood vessel of a living being of claim 13 and the computer program for executing a method according to claim 12 or 13 of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1A shows an embodiment of a blood flow measurement system for measuring a blood flow quantity inside a blood vessel of a living being using an intravascular blood flow measurement device;
Fig 1B shows a cross sectional view of an intravascular blood flow measurement device shown in Fig. 1A, as placed inside a blood vessel;
Fig. 2A shows the embodiment of a blood flow measurement system using the intravascular blood flow measurement device shown in Fig. 1A being expanded to a larger size;
Fig 2B shows a cross sectional view of the intravascular blood flow measurement device shown in Fig. 2A, as placed inside a blood vessel;
Fig. 3A shows another embodiment of a blood flow measurement system for measuring a blood flow quantity inside a blood vessel of a living being that comprises another embodiment of an intravascular blood flow measurement device;
Fig 3B shows a cross sectional view of the intravascular blood flow measurement device shown in Fig. 3A, as placed inside a blood vessel;
Fig. 4 shows four pressure curves obtained by measuring the pressure of blood flow in a direction perpendicular to the main direction of blood flow over a predetermined time span covering one heart cycle and plotted versus a normalized and shifted heart cycle time.
Fig. 5 shows an example of curve fitted using the measured pressure values shown in Fig. 4, and plotted as a function of maximum values of the cross sectional areas of the expandable body section at which they were measured;

and

Fig. 6 shows a flow diagram of an embodiment of a method for controlling operation of an intravascular blood flow measurement device.

Fig. 7 shows a flow diagram of an embodiment of a method for determination of a blood flow quantity inside a blood vessel of a living being.

DETAILED DESCRIPTION OF EMBODIMENTS

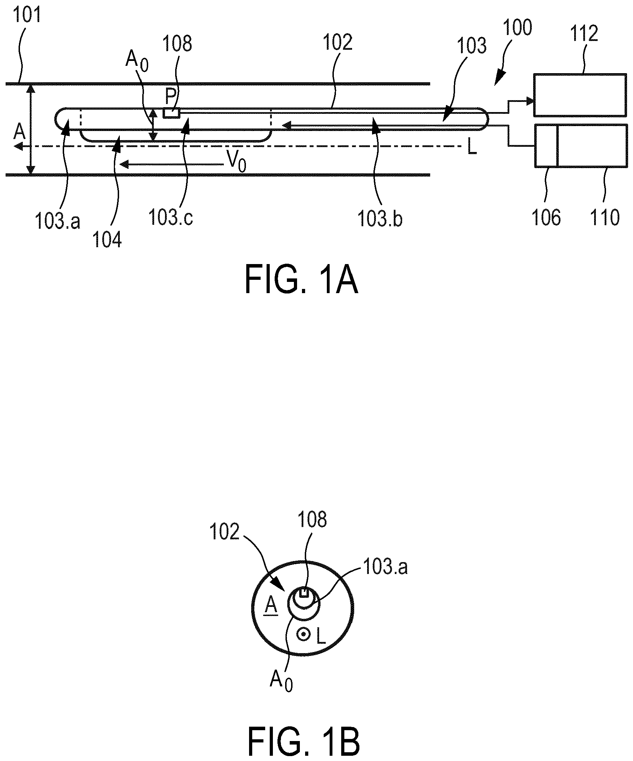

Fig. 1A shows an embodiment of a blood flow measurement system 100 for measuring a blood flow quantity inside a blood vessel 101 of a living being using an intravascular blood flow measurement device 102. Fig 1B shows a cross sectional view of the intravascular blood flow measurement device 102, as placed inside the blood vessel 101, having a value of cross sectional area given by A.

The blood vessel 101 has a cross sectional area value A as explicitly indicated in Fig 1B by an underlined letter A and indirectly indicated in Fig. 1A by a double arrow labeled A indicating the diameter of the blood vessel.

The intravascular blood flow measurement device 102 comprises a body 103 suitable for placement inside the blood vessel 101 of a living being and extending along a longitudinal direction L that substantially coincides with a main direction of blood flow if the measurement device is well aligned within the blood vessel.

The presence of the intravascular blood flow measurement device 102 in the vessel 101 alters a value of blood flow velocity. More specifically, the value of the blood flow velocity at a position P depends on an actual value of the cross sectional area of the intravascular blood flow measurement at the same longitudinal position P. The larger the cross sectional area of the intravascular blood flow measurement, the higher the blood flow velocity at that position, and the lower the pressure exerted by the blood flow on the longitudinal position P.

The intravascular blood flow measurement device 102 includes a body 103, e.g. a guidewire or a catheter that has fixed cross sectional area values along its longitudinal extension in a distal end section 103.a, a proximal end section 103.b, and in a middle section 103.c: The middle section is located between the distal and proximal end sections. The body 103 comprises an expandable body section 104 arranged at the middle section 103.c. The expandable body section 104 has a cross-sectional area perpendicular to the longitudinal direction that is gradually changing in the longitudinal direction L. Provided that the expandable body section presents a predetermined unexpanded size associated with a value of an unexpanded cross sectional area of the expandable body section in an unexpanded state, the expandable body section is controllably and reversibly expandable to assume a respective one of at least two different predetermined expanded sizes associated with at least two respective values of an expanded cross sectional area of the expandable body section. 104. Therefore, the expandable body section 104 is configured to adopt at least three different sizes, at least two of which are expanded sizes.

In the present embodiment, the expansion and contraction of the expandable body section 104 is achieved using an electro-active polymer (EAP). EAPs exhibit a change in size or shape when stimulated by a suitable electric drive signal, which herein is also referred to as the electrical expansion-control signal. The electro-active polymer controllably changes its size or shape in response to receiving the electrical expansion-control signal. Suitable EAPs include piezoelectric polymers, electromechanical polymers, relaxor ferroelectric polymers, electrostrictive polymers, dielectric elastomers, liquid crystal elastomers, conjugated polymers, ionic polymer-metal composites, ionic gels, polymer gels, etc.

In the state shown in Fig. 1A and Fig. 1B, the expandable body section 104 is currently set to a size associated with a value of a cross sectional area of $A_0$, as explicitly indicated in Fig 1B and indirectly referred to in Fig. 1A by the diameter of the expandable body section at the position P, which is shown by a double arrow that is also labeled $A_0$.

A controllable actuator 106 is arranged and configured to provide a suitable drive voltage to the expandable body section 104 to allow expansion one of the at least three sizes (for example either the unexpanded size or one of the at least two expanded sizes), in response to a corresponding size control signal. The size control signal is provided by the control unit 110.

In other embodiments (not shown), where the expansion and contraction of the expandable body section is achieved by using an inflatable balloon, the actuator 106 is configured to change the size by drive a volume expansion, in particular by pumping a pressurized fluid or gas into or out of the inflatable balloon, depending on whether an expansion or reduction of the volume is currently desired.

In this case, the blood flows inside the blood vessel 101 at the predetermined longitudinal position P with a blood flow velocity given by $v_0$.

A pressure sensor unit comprises, in this particular intravascular blood flow measurement device, a single pressure sensor 108 that is arranged on the body 103 within the longitudinal extension 103.c of the expandable body section 104 at a longitudinal position P. The pressure sensor 108 provides, for each of at least three values of cross sectional area of the body (that include the at least two values of the expanded cross-sectional area of the expandable body section), a respective sequence of pressure signals indicative of a pressure of blood flow in the blood vessel over a predetermined measuring time span defined with respect to a heart cycle period. The sequence of pressure signals are then provided to a flow determination unit 112.

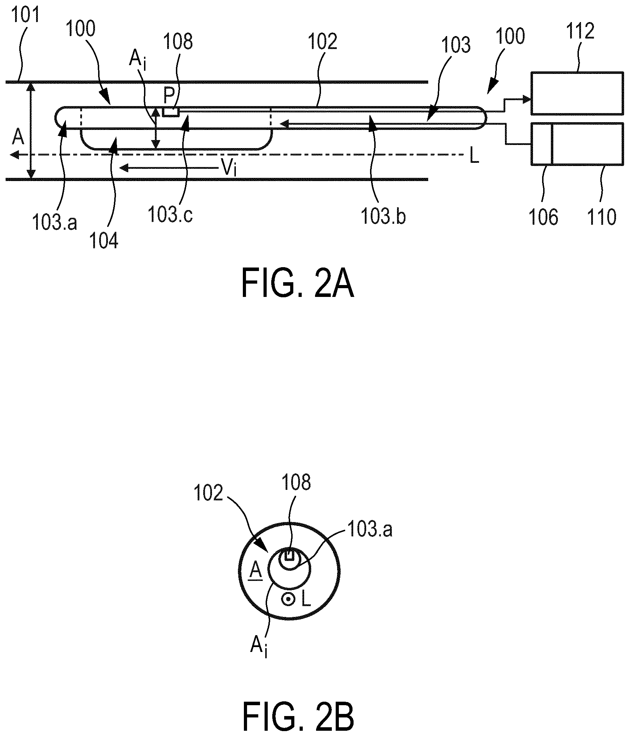

In the following, additional reference will be made to Figs. 2A and 2B. Fig. 2A shows the blood flow measurement system 100 using the intravascular blood flow measurement device 102 shown in Fig. 1A with an expanded size of the expandable body sections. Fig. 2B in turn shows a cross sectional view of the intravascular blood flow measurement device 102 shown in Fig. 2A, as placed inside a blood vessel having a cross sectional area value of A.

Thus, Figs. 2A and 2B show intravascular blood flow measurement device 102 after having been set to a larger maximum cross-sectional value $A_i$. In this situation, the blood flows inside the blood vessel 101 at the predetermined longitudinal position P with an increased velocity given by $v_i$, thus creating a pressure drop at position P when compared to the situation described with reference to Figs. 1A and 1B.

The flow determination unit 112 is configured to calculate a value of a blood flow quantity Q as it will be explained in the following.

The calculation of the blood flow is performed by using the at least three values of cross sectional area of the body, including the at least two values of the expanded cross-sectional area of the expandable body section 104, the respective pressure signals associated with these values, taken at respective measuring times corresponding to an identical phase of the heart cycle, and a known value of a density of blood. For this calculation, Bernoulli's law is used.

Bernoulli's law states that for two different pressure values $p_0$ and $p_i$ and two corresponding different flow velocities $v_0$ and $v_i$ the following equation holds:

$$p_0 - p_i = \frac{\rho}{2}\left(v_i^2 - v_0^2\right) \tag{5}$$

Assuming that the three different sizes of the expandable body section are allocated to cross sectional area values of $A_0$, $A_1$ and $A_2$, which result in blood flow velocities at the position P of the pressure sensor having values $v_0$, $v_1$ and $v_2$ respectively, and that the corresponding sequences of pressure signals indicative of a pressure of blood flow in the blood vessel over a predetermined measuring time span defined with respect to a heart cycle period are given by $p_0(t)$, $p_1(t)$ and $p_2(t)$, the value of a blood flow quantity Q can be calculated from one of the following equations:

$$\left(A - A_0\right)v_0 = \left(A - A_1\right)v_1 = \left(A - A_2\right)v_2 = Q \tag{6}$$

In order to solve any of these equations, the value of A and the value of one of the blood flow velocities have to be determined.

For that, a system of two equations can be obtained from the previous equations:

$$p_1 = \frac{v_0^2 \rho}{2}\left(1 - \left(\frac{A - A_0}{A - A_1}\right)^2\right) + p_0 \tag{7.1}$$

$$p_2 = \frac{v_0^2 \rho}{2}\left(1 - \left(\frac{A - A_0}{A - A_2}\right)^2\right) + p_0 \tag{7.2}$$

This system can be resolved to obtain the values of A and $v_0$. Alternatively, other sets of values would lead to corresponding systems of equations that would result in values of A and $v_1$, or A and $v_2$.

Once these values are determined, they can be used as an input for the matching equation (6) to obtain the value of the blood flow quantity Q.

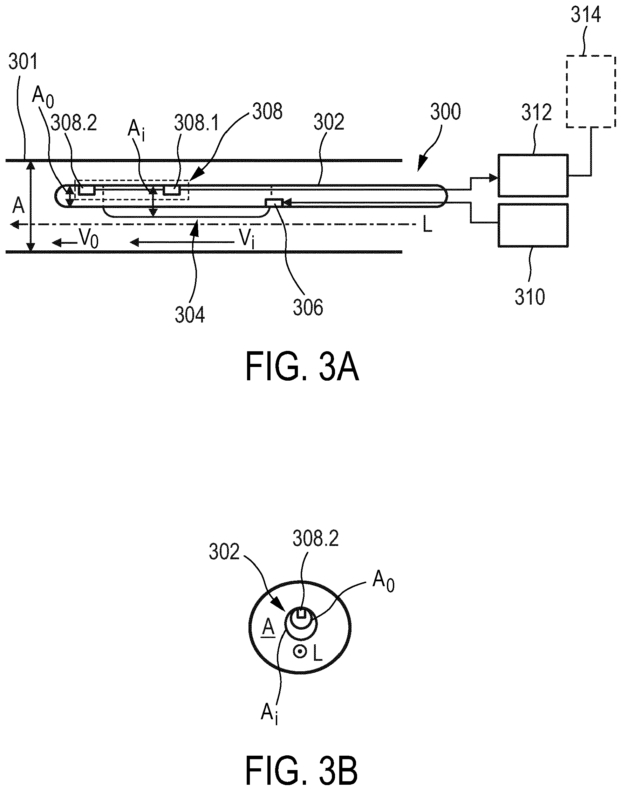

Fig. 3A shows another embodiment of a blood flow measurement system 300 for measuring a blood flow quantity inside a blood vessel 301. The blood flow measurement system 300 comprises another embodiment of an intravascular blood flow measurement device 302. Fig 3B shows a cross sectional view of the intravascular blood flow measurement device shown in Fig. 3A, as placed inside a blood vessel 301.

Fig. 3A shows an intravascular blood flow measurement device 302 that closely resembles the embodiment of Figs. 1 and 2. The following description focuses on features distinguishing the present embodiment from that described earlier. The intravascular blood flow measurement device 302 comprises a pressure sensor unit 308 comprising two pressure sensors 308.1 and 308.2. The pressure sensor 308.1 is arranged on the body 302 within the longitudinal extension of the expandable body section 304, whereas pressure sensor 310.2 is arranged on the body 302 outside the longitudinal extension of the expandable body section 304. The size of the expandable body section is set by the controllable actuator 306 in response to a corresponding size control signal provided by the control unit 310.

The flow determination unit 312 receives sequences of pressure signals from pressure detectors 308.1 and 308.2, and is also configured to determine the value of the blood flow quantity inside the blood vessel 102.

The expandable body section 304 is controllably and reversibly expandable to assume a respective one of at least two different predetermined sizes associated with at least two respective values of a cross sectional area of the expandable body section. The two different predetermined sized can be one non-expanded size and an expanded size or two expanded sizes. The controllable actuator 306 is configured to set the expandable body section to one of the at least two sizes. The pressure sensor unit 308 is configured to provide, for each of at least three values of cross sectional area of the body 302, a respective sequence of pressure signals indicative of a pressure of blood flow in the blood vessel over a predetermined measuring time span defined with respect to a heart cycle period. In the present case, two of the sequences are provided by pressure sensor unit 308.1 at two different associated sizes of the expandable body section 304, whereas the third sequence is provided by sensor 308.2 at a fixed size associated with the cross sectional area value of the body outside the longitudinal extension and given by $A_0$.

The intravascular blood flow measurement device 302 thus simultaneously provides two pressure signals obtained at two different longitudinal positions of the measuring head body that have different cross sectional area values ($A_0$ and $A_i$ in Fig. 3B). The intravascular blood flow measurement device 302 is therefore more robust against variations between heart cycles, since two sequences of pressure values can be provided simultaneously to the flow determination unit.

The intravascular blood flow measurement device 302 forms, with the control unit 310 and the flow determination unit 312, a particular embodiment of a blood flow measurement system for measuring a blood flow quantity Q or the blood flow velocity $v_0$ according to eq. 1-3.

In operation, particular care has to be taken that the device itself does not limit blood flow. In some blood flow measurement systems, therefore, the evaluation unit is further configured to support safe operation. This can be done using a specific evaluation involving a determination of a fit-error measure. This evaluation is based on using the expandable body section assuming at least three different predetermined expanded sizes.

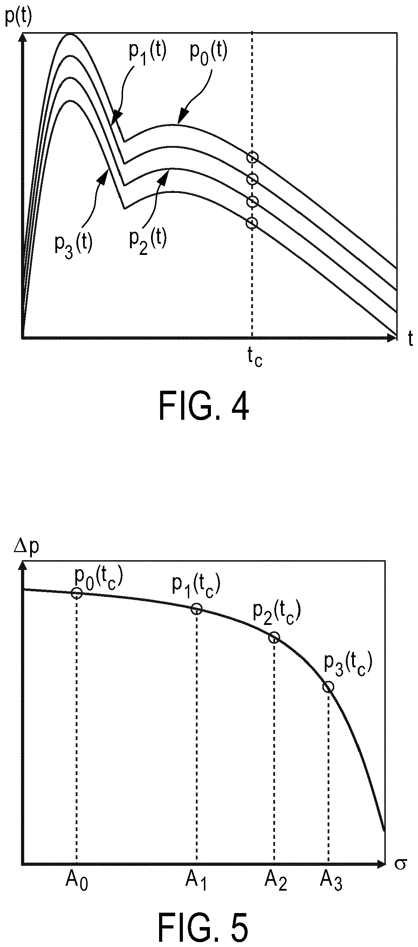

Details will be explained in the following with reference to Figs. 4 and 5. Fig. 4 shows four curves $p_0(t)$, $p_1(t)$, $p_2(t)$ and $p_3(t)$, obtained by measuring the pressure of blood flow in a blood vessel over a predetermined time span covering one heart cycle and plotted versus a normalized and shifted heart cycle time. Each curved is measured while the expandable body section has been set to adopt a different size, given by a value of a maximum cross sectional area at a predetermined longitudinal position of the measuring head body. According to Bernoulli's law, the curves measured at a higher cross-sectional value of the expandable body section show lower pressure values for any given relative time within a heart cycle (e.g. $t_c$) than those of the curves measured at lower cross-sectional area values.

The value of flow rate can be obtained by fitting the pressure values obtained at an identical normalized heart cycle time $t_c$, as it is shown in Fig 5. Fig. 5 shows an example of a fitted curved according to eq. 4 and using the values $p_i(t_c)$ (for i=0, 1, 2, and 3) shown in Fig. 4 that are plotted versus the cross sectional area value $\sigma$ of the expandable body section at which they were measured ($A_i$, for i=0, 1 2, and 3). The values of the cross sectional area A of the vessel, and of the flow velocity $v_0$ are thus obtained and can be further used to calculate the flow rate Q according to eq. (6).

In terms of safe operation, the inventors have recognized that the general form of equations (7.1) or (7.2), given by equation (3) will only fit four or more pairs ($p_i$, $A_i$) of cross-sectional area and measured pressure if the flow of blood is not limited by the presence of the intravascular blood flow measurement device. Therefore, a quality value of the fit, such as, but not limited to the mean squared error, can be used as an indicator for measurement error.

In some embodiments, the blood flow measurement system 300 further comprises a user interface 314 that receives the error output signal and, upon receiving the error output signal, to provide output information to a user. The output information indicates that a blood flow limitation currently induced by the intravascular blood flow measurement device exceeds a predetermined maximum blood flow limitation amount in the blood vessel and thus allows adjusting the settings. Such adjustment can be made by manual control or automatically.

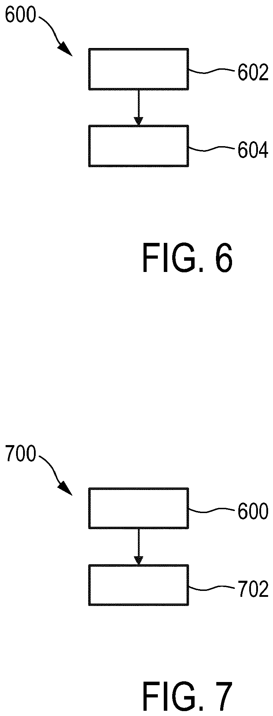

Fig. 6 shows a flow diagram of an embodiment of a method 600 for controlling operation of an intravascular blood flow measurement device. The method comprises a step 602 in which a size control signal indicative of a respective one of at least two predetermined expanded sizes to be assumed by the expandable body section of the body of the intravascular blood flow measurement device is sequentially provided to the controllable actuator of the intravascular blood flow measurement device. The method also comprises a step 604 in which a provision of sequence of pressure signals by a pressure sensor unit is controlled. The pressure sensor unit comprises at least one pressure sensor arranged on the body of the intravascular blood flow measurement device within a longitudinal extension of the expandable body section. The sequences of pressure signals are provided for each of at least three values of cross sectional area of the body, including the at least two values of the expanded cross-sectional area of the expandable body section. The sequences of pressure signals are indicative of a respective blood flow in the blood vessel over a predetermined measuring time span defined with respect to a heart cycle period.

Fig. 7 shows a flow diagram of a method 700 for determination of a blood flow quantity inside a blood vessel of a living being.

The method comprises performing the method 600 for controlling operation of an intravascular blood flow measurement device. Furthermore, the method 700 includes a step 702 in which a value of a blood flow quantity inside the blood vessel is calculated. For this calculation, the at least three values of cross sectional area of the body, the pressure signals associated with the at least three values of cross sectional area of the body and taken at respective measuring times corresponding to an identical phase of the heart cycle, and a known value of a density of blood are used.

Additionally, in particular embodiments of the method 700, step 702 includes using Bernoulli's law, the at least three values of cross sectional area of the body, the associated sequences of pressure signals, and a known value of a density of blood, solving a system of two equations having the form

$$p_i = \frac{v_0^2 \rho}{2}\left(1 - \left(\frac{A - A_0}{A - A_i}\right)^2\right) + p_0$$

and determining the blood flow quantity Q by calculating

$$\left(A - A_0\right)v_0 = Q$$

or any equivalent equation using a given value of the cross sectional area of the expandable body section and the respective value of the blood flood velocity.

In summary, thus, a blood flow measurement system for measuring a blood flow quantity inside a blood vessel includes an intravascular blood flow measurement device with a body that comprises an expandable body section. A control unit sequentially provides a size control signal indicative of a respective one of the at least two predetermined expandable sizes to be assumed by the expandable body section. A pressure sensor unit measures respective sequences of blood pressure signals over a measuring time span defined with respect to a heart cycle period. A flow determination unit uses the values of cross sectional area of the expandable body section, the associated pressure signals and a known value of a density of blood, to calculate a value of a blood flow quantity inside the blood vessel based on Bernoulli's law.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

**Claims**

1. An intravascular blood flow measurement device (102), comprising:

 - a body (103) suitable for placement inside a blood vessel (101) of a living being and extending along a longitudinal direction (L);
 - an expandable body section (104) of the body, having a longitudinal extension in the longitudinal direction and a cross-sectional area perpendicular to the longitudinal direction that is controllably and reversibly expandable to assume a respective one of at least two different predetermined expanded sizes associated with at least two respective values of an expanded cross sectional area of the expandable body section;
 - a controllable actuator (106) arranged and configured to set the expandable body section to one of the at least two sizes; and
 - a pressure sensor unit (108) comprising at least one pressure sensor arranged on the body within the longitudinal extension of the expandable body section, the pressure sensor unit being configured to provide, for each of at least three values of cross sectional area of the body, including the at least two values of the expanded cross-sectional area of the expandable body section, a respective sequence of pressure signals indicative of a pressure of blood flow in the blood vessel over a predetermined measuring time span defined with respect to a heart cycle period.

2. The intravascular blood flow measurement device of claim 1, wherein the body, including the expandable body section, has a stream-lined shape maintaining a laminar blood flow inside the blood vessel.

3. The intravascular blood flow measurement device of claim 1, wherein the expandable body section (104) comprises an electro-active polymer configured to controllably change its shape or volume in response to receiving an electrical expansion-control signal.

4. The intravascular blood flow measurement device of claim 1, wherein the expandable body section (104) comprises an inflatable balloon configured to controllably change its volume within a volume range, and wherein the controllable actuator is configured to controllably pump a pressurized fluid or gas into or out of the inflatable balloon.

5. The intravascular blood flow measurement device (300) of claim 1, wherein

   - the pressure sensor unit (308) comprises at least two pressure sensors (308.1, 308.2); and
   - at least one of the pressure sensors (308.2) is arranged on the measuring head body outside the longitudinal extension of the expandable body section (304).

6. A blood flow measurement system (100) for measuring a blood flow quantity inside a blood vessel (101) of a living being, the flow measurement system comprising:

   - an intravascular blood flow measurement device (102) according to claim 1;
   - a control unit (110) configured to sequentially provide to the controllable actuator (106) a size control signal indicative of a respective one of the at least two predetermined sizes to be assumed by the expandable body section; and
   - a flow determination unit (112), which is configured to receive the pressure signals provided by the pressure sensor unit (108) and, using the at least three values of cross sectional area of the body, the pressure signals associated with the at least three values of cross sectional area of the body and taken at respective measuring times corresponding to an identical phase of the heart cycle, and a known value of a density of blood, to calculate a value of a blood flow quantity inside the blood vessel.

7. The blood flow measurement system of claim 6, wherein

   - the pressure sensor unit comprises only one pressure sensor; and
   - the control unit is configured to provide to the controllable actuator a size control signal indicative of a respective one of at least three predetermined sizes to be assumed by the expandable body section.

8. The blood flow measurement system of claim 6, wherein the flow determination unit is configured, using Bernoulli's law, the at least three values of cross sectional area of the body, the associated sequences of pressure signals, and a known value of a density of blood, to solve a system of two or more equations, to determine a value of the cross sectional area of the blood vessel (A) and a value of a volume flow velocity ($v_0$) at a respective value of cross sectional area.

9. The blood flow measurement system of claim 8, wherein

   - the expandable body section is controllably and reversibly expandable to assume at least three different predetermined expanded sizes associated with at least three respective values of an expanded cross sectional area of the expandable body section;
   - the pressure sensor unit is configured to provide, for each of at least four values of cross sectional area of the body, including the at least three values of the expanded cross-sectional area of the expandable body section, the respective sequence of pressure signals;
   - the flow determination unit is further configured to determine, using a predetermined fitting model, calibration curve parameters fitting pressure values determined from the received pressure signals as a function of cross-sectional area of the body and to determine, using the pressure values and the associated values of the cross-sectional area of the body, a fit-error measure, to compare the fit-error measure with a pre-determined error threshold value, and, in case the fit-error measure exceeds the error threshold value, to provide an error output signal indicative thereof.

10. The blood flow measurement system (300) of claim 9, further comprising a user interface (314) configured to receive the error output signal and, upon receiving the error output signal, to provide output information to a user, the output information being indicative of a blood flow limitation induced by the intravascular blood flow measurement device exceeding a predetermined maximum blood flow limitation amount in the blood vessel.

11. The blood flow measurement system of claim 9, wherein the control unit is configured, in response to receiving the error output signal, to determine and provide to the controllable actuator corrected size control signals indicative of at least two corrected size values within a corrected size value range having a smaller corrected maximum size value in comparison with a maximum size value of the expandable body section set by the control unit before receiving the error output signal.

**Patentansprüche**

1. Ein Gerät zum Messen der intravaskulären Durchblutung (102), das Folgendes umfasst:

   - ein Gehäuse (103), das im Blutgefäß (101) eines Lebenswesens platziert und in Längsrichtung (L) erweitert werden kann;
   - einen erweiterbaren Bereich (104) des Gehäuses, der der Länge nach in Längsrichtung erweitert werden kann, sowie einen zur Längsrichtung lotrechten Querschnittsbereich, der gesteuert und reversibel erweitert werden kann, um eine von mindestens zwei vordefinierten erweiterten Größen einzunehmen, die mindestens zwei entsprechenden Werten für den erweiterten Querschnittsbereich des erweiterbaren Gehäusebereichs entsprechen;
   - ein steuerbarer Betätiger (106), der den erweiterbaren Gehäusebereich auf eine der mindestens zwei Größen bringt; und
   - eine Drucksensoreinheit (108), die mindestens einen an der längsgerichteten Erweiterung des erweiterbaren Gehäusebereichs des Gehäuses angebrachten Drucksensor umfasst, wobei die Drucksensoreinheit für jeden der mindestens drei Werte für den Querschnittsbereich des Gehäuses einschließlich der mindestens zwei Werte für den erweiterten Querschnittsbereich des erweiterbaren Gehäusebereichs jeweils eine Abfolge der Drucksignale bereitstellt, die den Durchblutungsdruck im Blutgefäß über die vordefinierte Messdauer angeben, und die anhand einer Herzzykluszeit definiert wurden.

2. Das Gerät zum Messen der intravaskulären Durchblutung gemäß Anspruch 1, wobei das Gehäuse einschließlich des erweiterbaren Gehäusebereichs über eine stromlinienförmige Form verfügt, um im Blutgefäß die laminare Durchblutung aufrechtzuerhalten.

3. Das Gerät zum Messen der intravaskulären Durchblutung gemäß Anspruch 1, wobei der erweiterbare Gehäusebereich (104) über ein elektroaktives Polymer verfügt, das so gesteuert werden kann, dass es seine Form oder sein Volumen ändert, wenn es ein elektrisches Steuersignal für die Erweiterung erhält.

4. Das Gerät zum Messen der intravaskulären Durchblutung gemäß Anspruch 1, wobei der erweiterbare Gehäusebereich (104) über einen aufblasbaren Ballon verfügt, der so gesteuert werden kann, dass er sein Volumen innerhalb eines Volumenbereichs. Zudem kann der steuerbare Betätiger so gesteuert werden, dass er eine unter Druck stehende Flüssigkeit oder ein Gas in den aufblasbaren Ballon pumpt.

5. Das Gerät zum Messen der intravaskulären Durchblutung (300) gemäß Anspruch 1, wobei:

   - die Drucksensoreinheit (308) über mindestens zwei Drucksensoren (308.1, 308.2) verfügt; und
   - mindestens einer der Drucksensoren (308.2) auf dem Messkopfgehäuse außerhalb der längsgerichteten Erweiterung des erweiterbaren Gehäusebereichs (304) angebracht ist.

6. Ein System zum Messen der Durchblutung (100) zum Messen der Durchblutungsmenge im Blutgefäß (101) eines Lebewesens, wobei das Durchblutungsmesssystem Folgendes umfasst:

   - ein Gerät zum Messen der intravaskulären Durchblutung (102) gemäß Anspruch 1;
   - eine Steuerungseinheit (110), die dem steuerbaren Betätiger (106) sequenziell ein Größensteuerungssignal bereitstellt, das jeweils eine der mindestens zwei vordefinierten Größen angibt, die der erweiterbare Gehäusebereich einzunehmen hat; und
   - eine Durchblutungsbestimmungseinheit (112), die die Drucksignale der Drucksensoreinheit (108) sowie anhand der mindestens drei Werte für den Querschnittsbereich des Gehäuses die Drucksignale empfängt, die den mindestens drei Werten für den Querschnittsbereich des Gehäuses zugeordnet und über die jeweilige Messdauer ermittelt wurden, die einer identischen Phase des Herzzyklus entspricht. Diese werden gemeinsam mit dem bekannten Wert für die Blutdichte verwendet, um einen Wert für die Durchblutungsmenge im Blutgefäß zu berechnen.

7. Das System zum Messen der Durchblutung gemäß Anspruch 6, wobei

   - die Drucksensoreinheit nur einen Drucksensor umfasst; und
   - die Steuerungseinheit die dem steuerbaren Betätiger ein Größensteuerungssignal bereitstellt, das jeweils eine der mindestens drei vordefinierten Größen angibt, die der erweiterbare Gehäusebereich einzunehmen hat.

8. Das System zum Messen der Durchblutung gemäß Anspruch 6, wobei die Durchblutungsbestimmungseinheit anhand der Bernoulli-Gleichung, der mindestens drei Werte für den Querschnittsbereich des Gehäuses, der entsprechenden Abfolge der Drucksignale und eines bekannten Werts für die Blutdichte ein System mit mindestens zwei Gleichungen löst, um einen Wert für den Querschnittsbereich des Blutgefäßes (A) sowie einen Wert für die Volumenströmungsgeschwindigkeit ($V_0$) für den entsprechenden Wert des Querschnittsbereichs zu bestimmen.

9. Das System zum Messen der Durchblutung gemäß Anspruch 8, wobei:

   - der erweiterbare Gehäusebereich gesteuert und reversibel erweitert werden kann, um mindestens drei verschiedene vordefinierte erweiterte Größen einzunehmen, die mindestens drei entsprechenden Werten für den erweiterten Querschnittsbereich des erweiterbaren Gehäusebereichs entsprechen;
   - die Drucksensoreinheit für jeden der mindestens vier Werte für den Querschnittsbereich des Gehäuses einschließlich der mindestens drei Werte für den erweiterten Querschnittsbereich des erweiterbaren Gehäusebereichs jeweils eine Abfolge der Drucksignale bereitstellt;
   - die Durchblutungsbestimmungseinheit zudem anhand eines vordefinierten Passungsmodells Kalibrierungskurvenparameter bestimmt, die zu den anhand der empfangenen Drucksignale als Funktion des Querschnittsbereichs des Gehäuses ermittelten Werten passen. Zudem wird anhand der Druckwerte und der entsprechenden Werte des Querschnittsbereichs des Gehäuses eine Passungsfehlermessung bestimmt, um diese mit einem vordefinierten Fehlerschwellenwert zu vergleichen und - sofern die Passungsfehlermessung den Fehlerschwellenwert überschreitet - ein entsprechendes Fehlerausgabesignal bereitzustellen.

10. Das System zum Messen der Durchblutung (300) gemäß Anspruch 9, das zudem eine Benutzerschnittstelle (314) umfasst, die das Fehlerausgabesignal empfängt und beim Empfangen des Fehlerausgabesignals Ausgabedaten für den Benutzer bereitstellt, die auf eine eingeschränkte Durchblutung hinweisen, die dadurch verursacht wird, dass das Gerät zum Messen der intravaskulären Durchblutung im Blutgefäß einen vordefinierten Höchstwert für die Durchblutung überschreitet.

11. Das System zum Messen der Durchblutung gemäß Anspruch 9, wobei die Steuerungseinheit beim Erhalt des Fehlerausgabesignals die korrigierten Größensteuerungssignale des steuerbaren Betätigers ermittelt und bereitstellt, um mindestens zwei korrigierte Größenwerte innerhalb eines Größenwertbereichs anzugeben, die im Vergleich zum von der Steuerungseinheit vor dem Erhalt des Fehlerausgabewerts festgelegten Höchstwert für die Größe des erweiterbaren Querschnittsbereichs über einen korrigierten kleineren Höchstwert für die Größe verfügen.

## Revendications

1. Dispositif de mesure d'écoulement sanguin intravasculaire (102), comprenant :

   - un corps (103) adapté à être placé à l'intérieur d'un vaisseau sanguin (101) d'un être vivant et s'étendant le long d'une direction longitudinale (L) ;
   - une section corporelle extensible (104) du corps, comportant une extension longitudinale dans la direction longitudinale et une zone de section transversale perpendiculaire à la direction longitudinale, laquelle est extensible de manière commandable et réversible pour assumer une taille respective des moins deux tailles expansées prédéterminées différentes associées à au moins deux valeurs respectives d'une zone de section transversale expansée de la section corporelle extensible ;
   - un actionneur commandable (106) agencé et conçu pour régler la section corporelle extensible à l'une des au moins deux tailles ; et

- une unité de capteur de pression (108) comprenant au moins un capteur de pression disposé sur le corps à l'intérieur de l'extension longitudinale de la section corporelle extensible, l'unité de capteur de pression étant conçue pour fournir, pour chaque valeur des au moins trois valeurs de la section transversale du corps, comprenant les au moins deux valeurs de la zone de section transversale extensible de la section corporelle extensible, une séquence respective des signaux de pression indiquant une pression de l'écoulement sanguin dans le vaisseau sanguin sur une période de temps de mesure prédéterminée définie par rapport à une période de cycle cardiaque.

2. Dispositif de mesure d'écoulement sanguin intravasculaire selon la revendication 1, dans lequel le corps, y compris la section corporelle extensible, présente une forme bordée de flux maintenant un écoulement sanguin laminaire à l'intérieur du vaisseau sanguin.

3. Dispositif de mesure d'écoulement sanguin intravasculaire selon la revendication 1, dans lequel la section corporelle extensible (104) comprend un polymère électro-actif conçu pour changer de manière commandable sa forme ou son volume en réponse à la réception d'un signal électrique de commande de l'expansion.

4. Dispositif de mesure d'écoulement sanguin intravasculaire selon la revendication 1, dans lequel la section corporelle extensible (104) comprend un ballonnet gonflable conçu pour changer de manière commandable son volume dans une plage de volumes, et dans lequel l'actionneur commandable est conçu pour pomper de manière commandable un fluide ou un gaz sous pression dans ou hors du ballonnet gonflable.

5. Dispositif de mesure d'écoulement sanguin intravasculaire (300) selon la revendication 1, dans lequel

   - l'unité de capteur de pression (308) comprend au moins deux capteurs de pression (308.1, 308.2) ; et
   - l'au moins un des capteurs de pression (308.2) est disposé sur un corps de tête de mesure à l'extérieur de l'extension longitudinale de la section corporelle extensible (304).

6. Système de mesure d'écoulement sanguin (100) pour mesurer une quantité d'écoulement sanguin à l'intérieur d'un vaisseau sanguin (101) d'un être vivant, ledit système de mesure d'écoulement comprenant :

   - un dispositif de mesure d'écoulement sanguin intravasculaire (102) selon la revendication 1 ;
   - une unité de commande (110) conçue pour fournir séquentiellement à l'actionneur commandable (106) un signal de commande de taille indicatif d'une taille respective parmi les au moins deux tailles prédéterminées à assumer par la section corporelle extensible ; et
   - une unité de détermination de l'écoulement (112), laquelle est conçue pour recevoir les signaux de pression fournis par l'unité de capteur de pression (108) et, à l'aide des au moins trois valeurs de la section transversale du corps, les signaux de pression associés à les au moins trois valeurs de la section transversale du corps et prises à des moments de mesure respectifs correspondant à une phase identique du cycle cardiaque et à une valeur connue d'une densité du sang, pour calculer une valeur d'une quantité d'écoulement sanguin à l'intérieur du vaisseau sanguin.

7. Système de mesure d'écoulement sanguin selon la revendication 6, dans lequel

   - l'unité de capteur de pression comprend un seul capteur de pression ; et
   - l'unité de commande est conçue pour fournir à l'actionneur commandable un signal de commande de taille indicatif d'une taille respective des au moins trois tailles prédéterminées à assumer par la section corporelle extensible.

8. Système de mesure d'écoulement sanguin selon la revendication 6, dans lequel l'unité de détermination de l'écoulement est conçue, à l'aide de la loi de Bernoulli, des au moins trois valeurs de la section transversale du corps, des séquences associées des signaux de pression et d'une valeur connue d'une densité de sang, pour résoudre un système d'au moins deux équations, pour déterminer une valeur de la section transversale du vaisseau sanguin (A) et une valeur d'une vitesse d'écoulement volumique ($v_0$) à une valeur respective de la zone de section transversale.

9. Système de mesure d'écoulement sanguin selon la revendication 8, dans lequel

- la section corporelle extensible est extensible de manière commandable et réversible pour prendre au moins trois tailles expansées prédéterminées différentes associées à au moins trois valeurs respectives d'une zone de section transversale expansée de la section corporelle extensible ;

- l'unité de capteur de pression est conçue pour fournir, pour chaque valeur des au moins quatre valeurs de la section transversale du corps, y compris les au moins trois valeurs de la zone transversale expansée de la section corporelle extensible, la séquence respective des signaux de pression ;

- l'unité de détermination de l'écoulement est en outre conçue pour déterminer, à l'aide d'un modèle de réglage prédéterminé, des paramètres de courbe d'étalonnage réglant les valeurs de pression déterminées à partir des signaux de pression reçus en fonction de la section transversale du corps et pour déterminer, à l'aide des valeurs de pression et des valeurs associées de la zone de section transversale du corps, une mesure d'erreur de réglage, pour comparer la mesure d'erreur de réglage avec une valeur seuil d'erreur prédéterminée et, dans le cas où la mesure d'erreur de réglage est supérieure à la valeur seuil d'erreur, pour fournir un signal de sortie d'erreur indicatif de celui-ci.

10. Système de mesure d'écoulement sanguin (300) selon la revendication 9, comprenant en outre une interface utilisateur (314) conçue pour recevoir le signal de sortie d'erreur et, lors de la réception du signal de sortie d'erreur, pour fournir des informations de sortie à un utilisateur, lesdites informations de sortie étant indicatives d'une limitation d'écoulement sanguin induite par le dispositif de mesure d'écoulement sanguin intravasculaire supérieure à une quantité maximale prédéterminée de limitation d'écoulement sanguin dans le vaisseau sanguin.

11. Système de mesure d'écoulement sanguin selon la revendication 9, dans lequel l'unité de commande est conçue, en réponse à la réception du signal de sortie d'erreur, pour déterminer et fournir à l'actionneur commandable des signaux de commande de la taille corrigée indiquant au moins deux valeurs de taille corrigées dans une plage de valeurs de taille corrigée comportant une valeur de taille maximale corrigée inférieure en comparaison avec une valeur de taille maximale de la section corporelle extensible définie par l'unité de commande avant de recevoir le signal de sortie d'erreur.

FIG. 1A

FIG. 1B

FIG. 2A

FIG. 2B

FIG. 3A

FIG. 3B

FIG. 4

FIG. 5

600

602

604

FIG. 6

700

600

702

FIG. 7

**REFERENCES CITED IN THE DESCRIPTION**

**Patent documents cited in the description**

- US 20150313478 A1

- US 5178153 A